# EUROPÄISCHE PATENTANMELDUNG

(11) **EP 0 878 533 A2**
(43) Veröffentlichungstag der Anmeldung: **18.11.1998**
(21) Anmeldenummer: 98250157.9
(22) Anmeldetag: 08.05.1998
(51) Int. Cl.: C10L 3/00, B01J 19/12

(54) **Verfahren und Vorrichtung zur photobiologischen Trennung von kohlendioxid- und methanhaltigen Gasgemischen**

(30) Priorität: 14.05.1997 DE 19721280
(71) Anmelder: Energy of Nature - Projektgesellschaft für umwelttechnische Anlagensysteme Leipzig mbH, 04109 Leipzig (DE)
(72) Erfinder: Bäzold, Dietmar, Dr., 99099 Erfurt (DE); Menschel, Claudia, Dr., 01187 Dresden (DE); Scharr, Sven, Dipl.-Ing., 04177 Leipzig (DE); Kretschmer, Andreas, Dipl.-Ing., 04720 Auterwitz (DE); Panning, Frank, Dr., 15366 Dahlwitz-Hoppegarten (DE)
(74) Vertreter: Ziebig, Marlene, Dr. Dipl.-Chem.

(57) **Zusammenfassung**

Die Erfindung betrifft ein Verfahren zur photobiologischen Trennung von kohlendioxid- und methanhaltigen Gasgemischen sowie eine Vorrichtung, die aus einem mit einer Lichtquelle versehenen und an die Gasleitung des Biogasreaktors (1) angeschlossenen Assimilator (2), in dem Algenbiomasse Kohlendioxid aus einem Gemisch brennbarer Gase aufnimmt, einem gegen Lichteinwirkung abgeschirmten Dissimilator (6), in dem die Algenbiomasse das Kohlendioxid wieder abgibt, und einem Photosynthesereaktor (9) besteht, der mit dem Kohlendioxid versorgt wird, wobei Assimilator (2) und Dissimilator (6) durch Rohrleitungen, die dem Transport der Algenbiomasse dienen, verbunden sind und der Dissimilator (6) mit dem Photosynthesereaktor (9) durch eine Rohrleitung zur Entnahme von Algenbiomasse verbunden ist.

## Beschreibung

Die Erfindung betrifft ein Verfahren und eine Vonichtung zur photobiologischen Trennung von kohlendioxidhaltigen Gemischen brennbarer Gase. Die Qualität kohlendioxidhaltiger Gemische brennbarer Gase, die als energiereiche Komponenten vornehmlich Methan und Wasserstoff enthalten, aus natürlichen Quellen oder aus biotechnologischen Fermentationsprozessen, wird durch die Entfernung von Kohlendioxid erheblich verbessert, so daß die Verwertung in Verbrennungsprozessen effizienter und umweltfreundlicher erfolgen kann oder die Nutzung solcher Gase in biotechnologischen Produktionsprozessen möglich wird. Dabei wird zur Abtrennung des Kohlendioxids von spezifischen Eigenschaften des Stoffwechsels von Algenbiomasse Gebrauch gemacht und die Energie, die zum Abtrennen des Kohlendioxids erforderlich ist, wird durch Photosynthese aus dem natürlichen Sonnenlicht gewonnen.

Es ist bekannt, daß photoautotrophe Algen die Eigenschaft haben, in wässrigen Medien bei adäquaten Milieubedingungen Kohlendioxid zum Aufbau von körpereigener Biomasse aufzunehmen. Zahlreiche Verfahren zur Erzeugung von Algenbiomasse, die in verschiedenster Weise verwertet werden kann, sind bekannt. In seinem Vortrag "Einige Aspekte des gegenwärtigen Standes und der zukünftigen Entwicklung der Biotechnologie der Mikroalgen", der beim "7th European Congress on Biotechnology" (19. - 23. Februar 1995) in Nizza gehalten wurde, gibt Dr. O.Pulz an, daß in den USA jährlich 3000 Tonnen Spirulina-Biomasse als Lebensmittel verkauft werden. Eine zunehmende Bedeutung wird die Produktion von Algenbiomasse erlangen, aus der bestimmte Wirkstoffe, beispielsweise Polysaccharide, Antioxidantien oder mehrfach ungesättigte Fettsäuren extrahiert werden. Die Gewinnung von Farbstoffen für das Färben von Lebensmitteln wird bereits in industriellem Maßstab betrieben. Darüberhinaus werden Algen auch zur Reinigung von Abwässern eingesetzt.

Ein Verfahren, das die Kultivierung von Mikroalgen zur Herstellung eines Futtermittels zum Einsatz bei der Schweinezucht beschreibt, wurde in DE 43 17 006 offenbart. Es werden verschiedene Inhaltstoffe erwähnt, die in Algen enthalten sein können und die Bedingungen für die Kultivierung beschrieben, die in offenen oder geschlossenen Systemen unter Kunst bzw. Naturlicht erfolgen kann. In DE 43 17 006 werden aber keine Angaben zur Quelle des Kohlendioxids und zum Umgang mit den Abgasen des Algenbioreaktors gemacht.

Es ist bekannt, daß die Produktivität der Algenbiomasse erhöht werden kann, wenn die Kohlendioxidkonzentration erhöht wird. Algenbioreaktoren werden deshalb mit Kohlendioxid versorgt, das entweder als technisches Gas in handelsüblicher Form vorrätig gehalten wird oder durch Verbrennung kohlenstoffhaltiger Brennstoffe bei Bedarf erzeugt wird. Die Bereitstellung des Kohlendioxids stellt einen nicht zu vernachlässigenden Kostenfaktor bei der Produktion von Algenbiomasse dar. Deshalb und auch weil die verwendeten Brennstoffe meist in Form fossiler Energieträgern eingesetzt werden, wird es als vorteilhafter betrachtet, wenn das Kohlendioxid aus den Abgasen von Kraftwerken zur Produktion von Algenbiomasse eingesetzt wird. Im Botanischen Institut der Universität Köln wird beispielsweise daran gearbeitet, durch die Nutzung von kohlendioxidhaltigen Abgasen aus Braunkohlekraftwerken den Treibhauseffekt zu mindern und Algenbiomasse zu produzieren. Dabei wird der von den Algen bei der Photosynthese freigesetzte Sauerstoff an die Umgebungsluft abgegeben. Als Quelle kohlendioxidhaltiger Gasströme wird auch die Abluft aerober biotechnischer Prozesse, beispielsweise die Kompostierung in geschlossenen Systemen vorgeschlagen. Es ist allerdings bisher kein Verfahren bekannt, bei dem das Kohlendioxid durch die Algenbiomasse in Form eines Gemischs brennbarer Gase dargeboten und das in seiner Zusammensetzung veränderte Gasgemisch einer Verwertung zugeführt wird.

Die Erfindung hat die Aufgabe, ein wirtschaftlich relevantes Verfahren zu schaffen, mit dem die bekannte Fähigkeit von Algen zur Aufnahme von Kohlendioxid, das diese im Prozeß der Photosynthese zu Sauerstoff und körpereigener Biomasse umwandeln, zur Abtrennung von Kohlendioxid aus Gasgemischen, die brennbare Gase in Form von Methan und Wasserstoff enthalten, genutzt wird. Die Aufgabe der Erfindung besteht darin, die Verwertbarkeit dieser Gase günstiger zu gestalten, da der Anteil an Kohlendioxid, der nicht zum energetischen Wert dieser Gase beiträgt, vermindert wird. Insbesondere besteht die Aufgabe der Erfindung darin, ein Verfahren und eine Vorrichtung bereitzustellen, die eine effektive Trennung CO₂/CH₄- haltiger Gasgemische erlauben, auch wenn die Gasgemische in solchen großen Mengen anfallen wie z.B. bei der industriellen anaeroben Vergärung organischen Materials.

Für die kohlendioxidhaltigen Gasgemische, aus denen nach dem erfindungsgemäßen Verfahren Kohlendioxid abgetrennt werden kann, gibt es natürliche Quellen, etwa in der Form von Ergasvorkommen niederer Qualität, sowie als Grubengas oder als Sumpfgas. Solche Gasgemische entstehen insbesondere als Produktgase biotechnischer Fermentationen, vornehmlich durch das Verfahren der anaeroben Vergärung biogener Stoffe. Für diese Gasgemische wird im allgemeinen zusammenfassend der Begriff "Biogas" verwendet, obwohl auch im Falle der anaeroben Faulung von Klärschlamm von "Faulgas" oder "Klärgas", bzw. bei Abfalldeponien von "Deponiegas" gesprochen wird.

Die Zusammensetzung derartiger Gasgemische kann in weiten Bereichen schwanken. Die Hauptbestandteile sind Methan und Kohlendioxid, aber die Konzentration der einzelnen Komponenten hängt der Art und Zusammensetzung des Ausgangsmaterials und der Betriebsweise des Fermentationssystems ab. Im günstigsten Falle können bei der anaeroben Umsetzung energiereicher Ausgangsmaterialien, etwa von Fettsäuren und Fetten, Methankonzentrationen von 75 - 80 % erreicht werden. Je weniger energiereich oder bioverfügbar die Ausgangsstoffe sind, desto mehr verringert sich der Anteil des Methans und vergrößert sich der Anteil des Kohlendioxids.

Bei der anaeroben Vergärung der als regenerative Energieträger besonders aussichtsreichen Kohlehydrate, wie Cellulose und Glucose, die auch als Abfallprodukte bei der Gewinnung nachwachsender Rohstoffe anfallen, kann im günstigsten Falle ein Verhältnis von Methan zu Kohlendioxid wie 1:1 erwartet werden. Das schränkt die möglichen stofflichen und energetischen Nutzungsvarianten dieses Gasgemisches ein und erhöht den technischen Aufwand zur Gewinnung und Verwertung des Methans. Technische Verfahren zur Abtrennung von Kohlendioxid aus Biogas sind seit langem bekannt und sind dadurch gekennzeichnet, daß sie einen hohen Bedarf an externer Energie aufiveisen, die in Form mechanischer Energie zum Komprimieren oder zum Abkühlen des Gasgemisches zugeführt werden muß und mit Hilfe eines elektrischen Antriebs realisiert wird. Ein erheblicher Teil der in den brennbaren Gasen enthaltenen Energie muß für das Abtrennen des Kohlendioxids aufgewandt werden.

Erfindungsgemäß wird die Abtrennung des Kohlendioxid durch photobiologische Behandlung des Gasgemisches bei Normaldruck und physiologischen Bedingungen durchgeführt. Die Energie zur Trennung der Gase wird in Form von Licht eingebracht, wobei vorzugsweise das natürliche Sonnenlicht genutzt wird.

Die Abtrennung des Kohlendioxids erfolgt in einem geschlossenen Photobioreaktor, in dem die Algenbiomasse in einer wässrigen Suspension mit dem Gasgemisch in Kontakt gebracht wird. Die Algen nehmen das Kohlendioxid durch Assimilation auf, fixieren im Prozeß der Photosynthese den darin enthaltenen Kohlenstoff in Form von Algenbiomasse und geben Sauerstoff ab, der zusammen mit den anderen Komponenten des Gasgemischs den Photobioreaktor verläßt.. Die brennbaren Komponenten des Gasgemischs, insbesondere Methan und Wasserstoff, sind in Wasser kaum löslich und zeigen geringe Wechselwirkungen mit dem Stoffwechsel der phototrophen Algenkulturen. Es können alle Ausführungsformen von geschlossenen Photobioreaktoren mit natürlicher oder künstlicher Beleuchtung verwendet werden.

Das Gasgemisch, das den Photobioreaktor verläßt, kann in einem Verbrennungsprozeß energetisch genutzt oder in einem biotechnologischen Verfahren unter Verwendung methanotropher Mikroorganismen eingesetzt werden. Durch die Tätigkeit der Algen wurde die Konzentration des Kohlendioxids vermindert und durch einen äquivalenten Anteil Sauerstoff ersetzt. Das Gasgemisch kann gleichermaßen als Brennstoff, wie als Grundstoff für die Kultivierung methanotropher Mikroorganismen eingesetzt werden. Beide Verwendungszwecke können parallel nebeneinander in beliebigem Verhältnis betrieben werden.

Bei der Verwertung des Gasgemischs in einem Verbrennungsprozeß weist das erfindungsgemäße Verfahren den Vorteil auf, daß durch die photobiologische Behandlung reiner Sauerstoff eingebracht wird, während bei Verwendung von Sauerstoff aus der Luft stets ungefähr 4/5 Stickstoff mit in den Verbrennungsprozeß eingebracht werden. Der Stickstoff aus der Luft ist maßgeblich verantwortlich für die Bildung von Stickoxiden, die aus Gründen des Umweltschutzes durch technische Maßnahmen, wie beispielsweise Abgaskatalysatoren, unter einem festgelegten Grenzwert bleiben müssen. Durch die photobiologische Behandlung eines Gasgemisches entsteht ein brennbares Gasgemisch, bei dem die Gefahr der Bildung von Stickoxiden erheblich reduziert ist.

Die überschüssige Algenbiomasse wird aus dem Photobioreaktor abgezogen und einer anaeroben Fermentation zugeführt, wo die leichtabbaubaren Anteile durch anaerobe Vergärung wieder in Biogas umgewandelt werden und die Menge des für weitere Verwendungszwecke zur Verfügung stehenden Methans erhöhen. Die schwerabbaubaren Biopolymere werden als Bodenverbesserungsmittel verwertet und fördern die Humusbildung. Nährstoffe, die für das Wachstum der Algen im Photobioreaktor erforderlich sind, werden durch Fest-Flüssigtrennung aus dem Ablauf der anaeroben Vergärung wiedergewonnen und dem Photobioreaktor zugeführt.

Die Algenbiomasse kann auch ganz oder teilweise als Lebensmittel oder Futtermittel verwertet werden und es können durch einen Aufarbeitungs- oder Extraktionsprozeß bestimmte Inhaltstoffe gewonnen werden, wobei die Rückstände vorteilhaft in der anaeroben Vergärung abgebaut werden und nicht als Abfallstoffe anfallen.

Eine besonders vorteilhafte Ausführungsform des erfindungsgemäßen Verfahrens ergibt sich, wenn die Eigenschaft der Algen genutzt wird, unter Streßbedingungen Kohlendioxid gasförmig aufzunehmen, in der Zelle zu speichern und gasförmig wieder freizusetzen, zum Beispiel, wenn sie Licht und Dunkelheit in kurzen Intervallen ausgesetzt werden. Hierzu wechselt ein Teil der Algenbiomasse in einem gesonderten Photoreaktorsystem in kurzen Intervallen zwischen einem Assimilator, und einem Dissimilator.

Erfindungsgemäß werden für den Assimilator und den Dissimilator geschlossene Reaktoren, die im folgenden als A/D-Reaktorsystem bezeichnet werden, verwendet, die es ermöglichen, daß nach Durchströmen des Assimilators ein an Kohlendioxid reduziertes Gasgemisch zur Verfügung gestellt wird, das einer Verwertung zugeführt werden kann, und daß dem Dissimilator das abgetrennte Kohlendioxid entnommen und einem Photobioreaktor zur Bildung von Algenbiomasse zugeführt werden kann. Im Photobioreaktor wird aus dem Kohlendioxid unter Einwirkung von Licht durch Photosynthese Sauerstoff freigesetzt, der bei der Verwertung des Gases aus dem Assimilator Verwendung findet. Das erfindungsgemäße Verfahren kann auch in der Form durchgeführt werden, daß das kohlendioxidhaltige Gasgemisch, wenn es nach Passieren eines Assimilators noch Kohlendioxid enthält, einem weiteren Assimilator zugeführt werden und auf diese Weise eine beliebige Anzahl von A/D-Reaktorsystemen in Reihe geschaltet werden kann.

Die Umsetzung des Kohlendioxids zu Biomasse erfolgt in einem gesonderten Photosynthesereaktor, der vorzugsweise die natürliche Sonnenstrahlung nutzt und dem das Kohlendioxid aus einem oder mehreren Dissimilatoren zugeleitet wird. Zur Anpassung an die tages- und jahrezeitlich veränderlichen Beleuchtungsverhältnisse kann das anfallende Kohlendioxid mit bekannten Einrichtungen zwischengespeichert werden kann und abgestimmt auf das natürliche Dargebot an Licht dem Photosynthesereaktor zugeführt werden.

Es ist für die Durchführung des erfindungsgemäßen Verfahrens vorteilhaft, wenn der Photosynthesereaktor nicht länger als 8 - 10 Stunden pro Tag der Dunkelheit ausgesetzt ist. Der Photosynthesereaktor kann deshalb mit Licht aus einer künstlichen Lichtquelle versorgt werden, wobei alle Arten von Lichtquellen in Frage kommen. Eine bevorzugte Ausführung besteht darin, das Licht mit leitfähigem Material in das Innere des Photosynthesereaktors zu führen. Dabei können insbesondere Materialien verwendet werden, die eine Filterwirkung für bestimmte Wellenlängen haben. Dadurch wird die Algenbiomasse zur Synthese von bestimmten Stoffen, z.B. von Farbstoffen stimuliert, die durch Extraktion gewonnen werden können.

### Ausführungsbeispiel

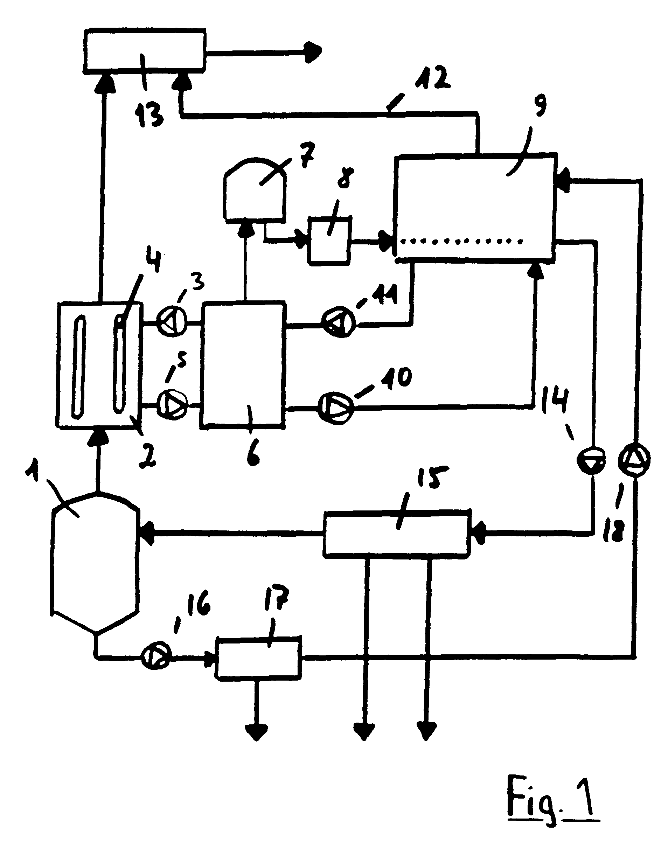

Eine zur Durchführung des erfindungsgemäßen Verfahrens geeignete Vorrichtung ist in Fig. 1 dargestellt. Das Gas, aus dem das Kohlendioxid abgetrennt werden soll, wird aus dem Biogasreaktor (1) entnommen. Im Biogasreaktor (1) werden organische Stoffe durch anaerobe Vergärung zu Biogas umgesetzt, das dem Assimilator (2) zugeführt wird. Der Assimilator (2) wird von Algenbiomasse durchströmt, die mit der Pumpe (3) zugeführt wird. Die Algenbiomasse wird im Assimilator (2) bei einer Beleuchtungsstärke von mindesten 500 Lux dazu angeregt, Kohlendioxid aufzunehmen und in den Zellen zu speichern.

Der Assimilator (2) ist mit einer Lichtquelle (4) in Form von Leuchtstoffröhren ausgestattet, die einen von der Tages- und Jahreszeit unabhängigen Betrieb gestatten. Algenbiomasse, die im Assimilator (2) Kohlendioxid aufgenommen hat, wird mit der Pumpe (5) in den Dissimilator (6) überführt. Da der Dissimilator (6) gegen Lichteinfall abgeschirmt ist, gibt die Algenbiomasse das aufgenommene Kohlendioxid wieder ab.

Das Kohlendioxid wird dem Gasbehälter (7) zugeführt, der als Zwischenspeicher für das Kohlendioxid dient, und aus dem es mit der Dosiereinrichtung (8) dem Photosynthesereaktor (9) zugeführt wird. Durch die Zwischenspeicherung des Kohlendioxids kann, solange es die natürlichen Lichtverhältnisse zulassen, auf eine künstliche Beleuchtung des Photosynthesereaktors verzichtet werden und das natürliche Sonnenlicht zum Aufbau von Algenbiomasse genutzt werden.

Im Photosynthesereaktor (9) wird das Kohlendioxid von den Algen vorwiegend mit Hilfe der natürlichen Sonnenstrahlung zum Aufbau von Biomasse genutzt. Gleichzeitig wird durch den Prozeß der Photosynthese Sauerstoff freigesetzt. Der Photosynthesereaktor (9) dient gleichzeitig zur Anzucht von Algenbiomasse, die durch das Umpumpen zwischen Assimilator (2) und Dissimilator (6) zum Abtrennen des Kohlendioxids benötigt wird. Da diese Algen einem hohen Streß ausgesetzt sind, wird ein Teilstrom mit der Pumpe (10) entnommen und dem Photosynthesereaktor (9) zugeführt und eine aliquote Menge frischer Algen wird durch Pumpe (11) aus dem Photosynthesereraktor (9) entnommen.

Der Sauerstoff, der im Photosynthesereaktor (9) entsteht wird durch die Leitung (12) der Gasvenvertungseinrichtung (13) zugeführt. In der Gasverwertungseinrichtung (13) wird das Abgas aus dem Assimilator (2), aus dem das Kohlendixid abgetrennt wurde, zur Gewinnung von Energie genutzt. Das Gas, dessen Heizwert durch das Passieren des Assimilators (2) erhöht wurde, wird im Verbrennungsmotor eines Blockheizkraftwerks genutzt. Der zur Verbrennung erforderliche Sauerstoff wird zumindest teilweise im Photosynthesereaktor (9) erzeugt und dieser Sauerstoff enthält nur unbedeutende Beimengungen anderer Gase.

Ein Teil der Abwärme des Blockheizkraftwerks wird zur Erwärmen des Biogasreaktors (1) und zum Erwärmen der Algenbiomasse im Photosynthesereaktor (9), im Assimilator (2) und im Dissimilator (6) genutzt. Der größte Teil der Abwärme steht für andere Verwendungszwecke zur Verfügung. Die elektrische Energie, die nicht für den Eigenbedarf der Anlage benötigt wird, kann an andere Verbraucher abgegeben oder ins Netz eingespeist werden.

Ein Teilstrom der Algenbiomasse, die im Photosynthesereaktor (9) wächst, wird mit der Pumpe (14) entnommen und der Aufbereitungseinrichtung (15) zugeführt. In der Aufbereitungseinrichtung (15) werden Algenbiomasse und Wasser getrennt, wobei das Wasser als biologisch gereinigtes Abwasser anfällt. Die entwässerte Algenbiomasse wird zur Gewinnung von wertvollen Inhaltstoffen (Farben, Vitamine) mit den für den jeweiligen Inhaltstoff spezifischen Extraktionsverfahren behandelt und die festen Reststoffe werden dem Biogasreaktor (1) zugeführt und darin zum Teil zu Kohlendioxid und Methan umgesetzt. Die nicht zu Biogas umsetzbaren Bestandteile aus der Algenbiomasse und aus den im Biogasreaktor (1) behandelten organischen Substanzen werden mit der Pumpe (16) der Fest-Flüssig-Trennung (17) zugeführt. Hier wird eine Flüssigkeit abgetrennt, die reich an mineralischen Nährstoffen ist, die von den Algen im Photosynthesereaktor zum Aufbau von Biomasse benötigt werden.

Mit der Pumpe (18) wird die Nährstofflösung wieder in den Photosynthesereaktor (9) zurückgeführt, wo die Nährstoffe von den Algen zum Aufbau von Biomasse verwendet werden.

Mit der beschriebenen Vorrichtung erfolgt die Verwertung der feuchten organischen Abfälle, die dem Biogasreaktor (1) zugeführt werden, wesentlich effizienter und umweltfreundlicher, als mit bekannten Einrichtungen.

Der Heizwert des im Biogasreaktor (1) entstehenden Gases wird durch die Abtrennung des Kohlendioxids erhöht. Im Photosynthesereaktor (9) wird reiner Sauerstoff erzeugt, der zur Verbrennung verwendet wird. Dadurch wird zur Verbrennung weniger oder gar keine Luft benötigt, und die Bildung von Stickoxiden während des Verbrennungsvorgangs vermindert. Im Photosyntheseraktor (9) entsteht unter Nutzung der Sonnenenergie aus dem Kohlendioxid und den Nährstoffen Algenbiomasse, die bei der Verwertung durch anaerobe Vergärung im Biogasreaktor (1) die Menge des Biogases erhöht. Durch die bei der Verwertung des aufbereiteten Gases in einem Blockheizkraftwerk entstehende Wärmeenergie können der Biogasreaktor (1), der Photosynthesereaktor (9), der Assimilator (2) und der Dissimilator (6) konstant auf einer Temperatur von ungefähr 25 bis 30° gehalten werden, bei der die biologischen Prozesse mit optimaler Geschwindigkeit ablaufen.

Im Biogasreaktor (1) einer landwirtschaftlichen Biogasanlage entstehen aus Gülle und organischen Abfällen täglich 1000 cbm Biogas, das sich aus 500 cbm Methan und 500 cbm Kohlendioxid zusammensetzt. Der Brennwert des Gases beträgt 5 kcal/cbm und wird auf 9 kcal/cbm erhöht.

Im Assilator (2) werden täglich 600 kg Kohlendioxid aus dem Biogas entnommen und im Dissimilator abgegeben. Das entspricht 200 kg Kohlenstoff, aus dem im Photosynthesereaktor (9) ca. 380 kg Algenbiomasse aufgebaut werden. Dabei werden 500 cbm Sauerstoff abgeben und zur Verbrennung des Methans eingesetzt. Bei Verbrennung des Gasgemisches, das den Assimilator (2) verläßt, mit Luft wären 4750 cbm Luft erforderlich, die 3850 cbm Stickstoff enthält. Durch den Einsatz photosynthetisch erzeugten Sauerstoffs sind nur 2375 cbm Luft erforderlich, das heißt die Stickoxidbildung reduziert sich um etwa 50 %.

Die Algenbiomasse wird im Biogasreaktor (1) zu Biogas umgesetzt und liefert ungefähr 330 cbm Biogas, das 165 cbm Methan enthält. Das sind zusätzlich 1650 kWh/d, die durch Photosynthese gewonnen werden.

### Liste der Bezugszeichen

1. Biogasreaktor
2. Assimilator
3. Speisepumpe Assimilator
4. Lichtquelle Assimilator
5. Speisepumpe Dissimilator
6. Dissimilator
7. Zwischenspeicher für Kohlendioxid
8. Dosiereinrichtung für Kohlendioxid
9. Photosynthesereaktor
10. Speisepumpe Photosynthesereaktor
11. Speisepumpe für frische Algen
12. Abführung des photosynthetisch erzeugten Sauerstoffs
13. Einrichtung zur Gasverwertung
14. Speisepumpe für die Aufbereitung der Algenbiomasse
15. Aufbereitung der Algenbiomasse
16. Speisepumpe Fest-Flüssig-Trennung
17. Fest-Flüssig-Trennung
18. Pumpe für Nährstofflösung

## Patentansprüche

1. Verfahren zur photobiologischen Trennung von kohlendioxid- und methanhaltigen Gasgemischen durch Umwandlung des Kohlendioxids mittels Photosynthese in Algenbiomasse und Sauerstoff,
dadurch gekennzeichnet,
daß das Kohlendioxid aus dem Gasgemisch durch lichtinduzierte Assimilation und anschließende Dissimilation einer Algenkultur entfernt wird, wobei die Algenkultur in kurzen Abständen zwischen einem Zustand, in dem sie unter Lichteinwirkung Kohlendioxid aufnimmt und einem Zustand der Dunkelheit, in dem sie das Kohlendioxid abgibt, wechselt und anschließend das bei der Dissimilation freigesetze Kohlendioxid in einem Photosynthesereaktor durch Algenkulturen in Algenbiomasse und Sauerstoff umgewandelt wird.

2. Verfahren nach Anspruch 1,
dadurch gekennzeichnet,
daß Assimilation und Dissimilation in mehreren hintereinandergeschalteten Reaktoren durchgeführt werden.

3. Verfahren nach Anspruch 1 oder 2,
dadurch gekennzeichnet,
daß das bei der Dissimilation freigesetzte Kohlendioxid in einem Speicher gesammelt und dem Photosynthesereaktor zur Ausnutzung der natürlichen Sonnenstrahlung nur zeitweise zugeführt wird.

4. Verfahren nach einem der Ansprüche 1 bis 3,
dadurch gekennzeichnet,
daß ein Teilstrom der Algenbiomasse dem Photosynthesereaktor entnommen und zum Zwecke der lichtinduzierten Assimilation und Dissmilitation in einem aus Assimilator und Dissimilator bestehenden Reaktorsystem verwendet und wieder dem Photosynthesereaktor zugeführt wird.

5. Verfahren nach einem der Ansprüche 1 bis 4,
dadurch gekennzeichnet,
daß das nach Abtrennung von Kohlendioxid verbleibende Gasgemisch wenigstens teilweise einem Verbrennungsprozeß zugeführt wird.

6. Verfahren nach einem der Ansprüche 1 bis 5,
dadurch gekennzeichnet,
daß das nach Abtrennung von Kohlendioxid verbleibende Gasgemisch wenigstens teilweise zur Kultivierung von methanotrophen Mikroorganismen genutzt wird.

7. Verfahren nach einem der Ansprüche 1 bis 6,
dadurch gekennzeichnet,
daß die unter Verwendung des abgetrennten Kohlendioxids photosynthetisch aufgebaute Algenbiomasse ganz oder teilweise zur Ernährung von nicht photosynthetisch aktiven Organismen verwendet wird.

8. Verfahren nach einem der Ansprüche 1 bis 7,
dadurch gekennzeichnet,
daß aus der unter Verwendung des abgetrennten Kohlendioxids photosynthetisch aufgebauten Algenbiomasse Wertstoffe extrahiert und die Extraktionsrückstände in einer anaeroben Vergärung zu Biogas umgesetzt werden.

9. Verfahren nach einem der Ansprüche 1 bis 6,
dadurch gekennzeichnet, daß
die gesamte unter Verwendung des abgetrennten Kohlendioxids photosynthetisch aufgebaute Algenbiomasse in einer anaeroben Vergärung zu Biogas umgesetzt wird.

10. Vorrichtung zur photobiologischen Trennung von kohlendioxid- und methanhaltigen Gasgemischen,
dadurch gekennzeichnet,
daß sie besteht aus einem mit einer Lichtquelle versehenen und an die Gasleitung des Biogasreaktors (1) angeschlossenen Assimilator (2), in dem Algenbiomasse Kohlendioxid aus dem Gasgemisch aufnimmt, einem gegen Lichteinwirkung abgeschirmten Dissimilator (6), in dem die Algenbiomasse das Kohlendioxid wieder abgibt, und einem Photosynthesereaktor (9), der mit dem Kohlendioxid versorgt wird, wobei Assimilator (2) und Dissimilator (6) durch Rohrleitungen, die dem Transport der Algenbiomasse dienen, verbunden sind und der Dissimilator (6) mit dem Photosynthesereaktor (9) durch eine Rohrleitung zur Entnahme von Algenbiomasse verbunden ist.

11. Vorrichtung nach Anspruch 10,
dadurch gekennzeichnet,
daß der Dissimilator (6) mit einem Zwischenspeicher (7) für das freigesetzte Kohlendioxid verbunden ist.

12. Vorrichtung nach Anspruch 10 oder 11,
dadurch gekennzeichnet,
daß dem Photosynthesereaktor (9) eine Dosiereinheit (8) zum Zudosieren des Kohlendioxids vorgeschaltet ist.

13. Vorrichtung nach einem der Ansprüche 10 bis 12,
dadurch gekennzeichnet,
daß der Dissimilator (6) und der Photosynthesereaktor (9) durch eine Rohrleitung zur Rückführung von Algenbiomasse verbunden sind.

14. Vorrichtung nach einem der Ansprüche 10 bis 13,
dadurch gekennzeichnet,
daß der Photosynthesereaktor (9) über eine Rohrleitung mit einer Pumpe (14) zum Fördern der Algenbiomasse mit einer Einrichtung zum Aufbereiten (15) der Algenbiomasse verbunden ist, die ihrerseits mit dem Biogasreaktor (1) verbunden ist, an den eine Fest-Flüssig-Trennung (17) angeschlossen ist, die zur Rückführung der Flüssigkeit ihrerseits durch eine Rohrleitung mit einer Pumpe mit dem Photosynthesereaktor (9) verbunden ist.

15. Vorrichtung nach einem der Ansprüche 10 bis 14,
dadurch gekennzeichnet,
daß der Assimilator (2) zur Überleitung des im Kohlendioxidgehalt verminderten Gases, ebenso wie der Photosynthesereaktor (9) zur Überleitung des entstandenen Sauerstoffs mit einer Einrichtung zur Gasverwertung (13) verbunden sind.
